# EUROPÄISCHE PATENTSCHRIFT

(11) **EP 2 500 096 B1**
(45) Veröffentlichungstag und Bekanntmachung des Hinweises auf die Patenterteilung: **18.07.2018**
(21) Anmeldenummer: 12158003.9
(22) Anmeldetag: 05.03.2012
(51) Int. Cl.: B01L 3/00, G01N 33/86, C12Q 1/56

(54) **VORRICHTUNGEN UND VERFAHREN ZUR BESTIMMUNG DER PLÄTTCHENFUNKTION IN EINEM ZENTRIFUGALANALYZER**
METHOD AND DEVICES FOR DETERMINING THE PLATELET FUNCTION IN A CENTRIFUGAL ANALYSER
DISPOSITIFS ET PROCÉDÉ DE DÉTERMINATION DE LA FONCTION PLAQUETTAIRE DANS UN ANALYSEUR CENTRIFUGE

(30) Priorität: 15.03.2011 EP 11158195
(43) Veröffentlichungstag der Anmeldung: 19.09.2012
(73) Patentinhaber: Siemens Healthcare Diagnostics Products GmbH, 35041 Marburg (DE)
(72) Erfinder: Endermann, Joerg, 35075 Gladenbach (DE); Zander, Norbert, 35039 Marburg (DE); Rechner, Andreas, 35041 Marburg (DE)

(56) Entgegenhaltungen:
- EP-A1- 0 797 097
- EP-A1- 1 693 109
- WO-A1-97/34698
- KRISCHEK BORIS ET AL: "Role of the retention test Homburg in evaluating platelet hyperactivity and in monitoring therapy with antiplatelet drugs", SEMINARS IN THROMBOSIS AND HEMOSTASIS, STUTTGART, DE, Bd. 31, Nr. 4, 1. August 2005 (2005-08-01) , Seiten 458-463, XP008108614, ISSN: 0094-6176

## Beschreibung

Die vorliegende Erfindung liegt auf dem Gebiet der Gerinnungsdiagnostik, genauer auf dem Gebiet der Plättchenfunktionsdiagnostik und betrifft Vorrichtungen sowie Verfahren zur Bestimmung der Plättchenfunktion in einem Zentrifugalanalyzer.

Physiologische Vorgänge, die einerseits die Fluidität des Blutes im Gefäßsystem gewährleisten und andererseits durch die Bildung von Blutgerinnseln die Vermeidung von extravasalen Blutverlusten sicherstellen, werden unter dem Begriff Hämostase zusammengefasst. An der Regulation der Hämostase sind eine Vielzahl von Proteinfaktoren beteiligt sowie auch zelluläre Komponenten, wie z.B. die Thrombozyten (Plättchen). Im Falle einer Gefäßverletzung kommt es zuerst zur Anlagerung von Thrombozyten an das subendotheliale Kollagen. Diese Adhäsion wird durch Adhäsivproteine, wie den von-Willebrand-Faktor (VWF) vermittelt. Während des Adhäsionsvorgangs werden die Thrombozyten aktiviert und schütten Mediatoren aus ihren Granulae aus, wodurch die Aggregation weiterer Thrombozyten sowie eine Verstärkung der Aktivierung induziert wird. Auf diese Weise erfolgt ein primärer Gefäßwandverschluss (primäre Hämostase), der erst durch weitere Reaktionen des plasmatischen Gerinnungssystems (sekundäre Hämostase) stabilisiert wird. Fehlregulationen dieser Prozesse können zu einer Thromboseneigung oder einer Blutungsneigung führen und je nach Schweregrad lebensbedrohliche Folgen haben.

In der Gerinnungsdiagnostik wurden verschiedene in vitro Testverfahren entwickelt, mit deren Hilfe bestimmt werden kann, ob das Blut eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Eine wichtige Teilfunktion des Gerinnungssystems, die gezielt untersucht werden kann, ist die primäre Hämostase, die wesentlich von der Funktionsfähigkeit der Thrombozyten abhängt.

Die Bestimmung der Plättchen- oder Thrombozytenfunktion ist eine klassische Aufgabe in der Hämostasediagnostik, die in einer Vielzahl klinischer Situationen wichtig ist, z.B. bei der Früherkennung kardiovaskulärer Erkrankungen, zur Diagnose von erworbenen oder angeborenen Thrombozytenfunktionsstörungen, zum Ausschluss von Blutungskomplikationen vor operativen Eingriffen oder zur Überwachung antithrombotischer Therapien. Medikamente, die die Aggregation von Thrombozyten hemmen, werden vorwiegend zur Prophylaxe und Therapie arterieller thromboembolischer Ereignisse, wie Myokardinfarkt oder Schlaganfall eingesetzt. Die verbreitetsten Wirkstoffe mit thrombozytenaggregationshemmender Wirkung sind Acetylsalicylsäure (ASS, Aspirin®) und die Thienopyridine Clopidogrel und Ticlopidin.

Im Stand der Technik sind verschiedene Verfahren zur Untersuchung der Thrombozytenfunktion bekannt. Bei der Blutungszeitbestimmung handelt es sich um einen in vivo-Globaltest, der die primäre Hämostase erfasst. Die Blutungszeit wird bestimmt, indem dem Patienten eine kleine Schnitt- oder Stichverletzung zugefügt wird und die Zeit bis zum Blutungsstillstand gemessen wird. Es handelt sich um einen schwer standardisierbaren, grob informativen Test, der vor allem in Notfallsituationen angewendet wird, um einen Überblick über die primäre Hämostase zu erhalten. Die Einnahme von Thrombozytenaggregationshemmern führt zu einer Verlängerung der Blutungszeit. Nachteilig an der Blutungszeitbestimmung ist, dass bei einer normalen Blutungszeit eine Thrombozytenfunktionsstörung nicht ausgeschlossen werden kann.

Verschiedene in vitro-Verfahren ermöglichen einen wesentlich empfindlicheren Nachweis von Thrombozytenfunktionsstörungen. Bei diesen Verfahren wird üblicherweise in einer Vollblutprobe oder in einer Probe von plättchenreichem Plasma (PRP) die Aggregation der Thrombozyten durch Zugabe eines Aktivators und/oder durch die Ausübung von Scherkräften induziert und die Aggregationsreaktion gemessen. Die meistverwendeten Aktivatoren, die zur Induktion der Thrombozytenaggregation verwendet werden, sind: ADP (Adenosin-5'-diphosphat), Kollagen, Epinephrin (Adrenalin), Ristocetin und verschiedene Kombinationen davon sowie Thrombin, TRAP (Thrombin-Receptor-Activating-Protein) oder Serotonin. Zur Ausübung von Scherkräften in vitro, die ein wichtiger Auslöser der Thrombozytenaggregation in vivo sind, werden unterschiedliche Verfahren angewandt, wie z.B. das Rühren der Plättchenprobe oder das Durchleiten oder Durchdrücken der Plättchenprobe durch Kanülen oder Aperturen mit geringem Durchmesser.

Bei der klassischen Lichttransmissionsaggregometrie (LTA), die auch als Plättchenaggregation nach Born bezeichnet wird, wird die Aggregationsfähigkeit der Thrombozyten im plättchenreichen Plasma in Gegenwart von aggregationsinduzierenden Substanzen in einem Aggregometer photometrisch gemessen. Durch die Aggregatbildung wird die Lichtdurchlässigkeit der PRP-Probe erhöht, so dass durch die Messung der Lichtdurchlässigkeit z.B. die Geschwindigkeit der Aggregatbildung bestimmt werden kann. Mit Hilfe der Lichttransmissionsaggregometrie können auch therapeutische Effekte von medikamentös eingesetzten Thrombozytenaggregationshemmern erfasst werden. Ein Nachteil der Lichttransmissionsaggregometrie ist, dass ausschließlich plättchenreiches Plasma als Probenmaterial verwendet werden kann. Plättchenreichem Plasma fehlen nicht nur wichtige Bestandteile des Blutes, wie z.B. die roten und weißen Blutkörperchen, sondern es erfordert außerdem eine zeitaufwändige und fehleranfällige Probenvorbereitung.

Das VerifyNow® System (Accumetrics) ist eine Weiterentwicklung der Lichttransmissionsaggregometrie, die die Untersuchung der Thrombozytenfunktion in Vollblutproben ermöglicht. In diesem System wird die Aggregationsreaktion der Thrombozyten durch die Zugabe von Fibrinogenbeschichteten Mikropartikeln verstärkt.

Ein völlig anderes Testprinzip zur Bestimmung der Thrombozytenfunktion ist im Platelet Function Analyzer (PFA-100®, PFA-200 Siemens Healthcare Diagnostics) verwirklicht. Es handelt sich dabei um einen globalen, automatisierten und standardisierten in vitro-Vollbluttest, bei dem die primäre Hämostase unter Flussbedingungen und damit in Gegenwart von hohen Scherkräften gemessen wird. Zur Simulation der Flussbedingungen und der Scherkräfte, wie sie in kleineren arteriellen Blutgefäßen herrschen, wird in einer speziellen Messzelle ein Unterdruck von etwa -40mbar erzeugt und das Citratvollblut, dass sich in einem Probenreservoir befindet, strömt durch eine Kapillare, die einen Durchmesser von etwa 200 µm hat. Die Kapillare mündet in eine Messkammer die mit einem Trennelement, z.B. einer Membran abgeschlossen ist, welche eine kapillare zentrale Öffnung (Apertur) enthält, durch die das Blut aufgrund des Unterdrucks durchtritt. In den meisten Fällen ist die Membran, zumindest in dem Bereich um die Apertur mit einem oder mehreren Aktivatoren, die die Thrombozytenaggregation induzieren, versetzt, so dass das vorbeiströmende Blut mit den aggregationsinduzierenden Substanzen im Bereich der Apertur in Kontakt kommt. Infolge der induzierten Adhäsion und Aggregation der Thrombozyten bildet sich im Bereich der Apertur ein Thrombozytenpfropf (Thrombus), der die Membranöffnung verschließt und den Blutfluss stoppt. In diesem System wird die Zeit gemessen, die bis zum Verschluss der Membranöffnung benötigt wird. Diese sogenannte Verschlusszeit korreliert mit der Funktionsfähigkeit der Thrombozyten. Eine Messzelle zur Verwendung in einem Verfahren zu Bestimmung der Thrombozytenfunktion anhand der Verschlusszeit ist z.B. in WO 97/34698 beschrieben. Es werden z.B. Messzellen verwendet, die mit einer Membran ausgestattet sind, die mit Kollagen (Kol) und zusätzlich entweder mit ADP oder mit Epinephrin (Epi) beschichtet ist. Verschiedene Trennelemente sowie deren Herstellung und Verwendung sind z.B. in WO 96/00899 A1 beschrieben.

Ein wiederum anderes Testprinzip zur Bestimmung der Thrombozytenfunktion beruht auf der forcierten Passage von Blut oder plättchenreichem Plasma durch einen Filter.

Uchiyama, S. et al. (Thrombosis Research 31: 99-116, 1983) beschreiben den sog. Filter Bleeding Time (FBT) Test. Bei diesem Verfahren wird Vollblut unter konstantem Druck (ca. 150 mm Hg) durch einen Polyesterfaserfilter (Dacron®) geleitet. Plättchenaggregate verstopfen die Filterporen und vermindern die Durchflussrate. Die Blutungszeit FBT ist die Zeit, die vom Beginn des Durchflusses bis zu dem Zeitpunkt vergeht, in welchem die Durchflussrate unter weniger als ein Tropfen pro 30 Sekunden gefallen ist.

In GB 2175691 A ist eine Weiterentwicklung des FBT Tests nach Uchiyama et al. beschrieben. Hier wird eine Vollblutprobe mittels Überdruck durch einen Filter bestehend aus einem Fasergeflecht passagiert. Der Filter weist Poren unterschiedlicher Größe auf und erlaubt die Passage von Partikeln mit einem Durchmesser von bis zu 10 µm. Dies bewirkt, dass die Probe mit geringeren Drücken von nur 20 bis 100 mm Hg durch den Filter gedrückt werden kann. Größere Thrombozytenaggregate verstopfen die Poren und blockieren zunehmend den Durchtritt von Probenmaterial. Die Bestimmung der Durchflussrate bzw. der Vergleich der Plättchenanzahl im gefilterten Eluat mit der Plättchenanzahl in der ungefilterten Probe geben Aufschluss über die Aggregationsfähigkeit der Plättchen und damit über die Thrombozytenfunktion.

Ein anderes Verfahren zur Bestimmung der Thrombozytenfunktion, welches auf dem Prinzip der forcierten Passage von Blut oder plättchenreichem Plasma durch einen Filter beruht, ist der sog. Retention Test Homburg (RTH) (Krischek, B. et al., Seminars in Thrombosis and Hemostasis 31(4): 449-457, 2005; Krischek, B. et al., Seminars in Thrombosis and Hemostasis 31(4): 458-463, 2005). Bei diesem Verfahren wird Vollblut oder plättchenreiches Plasma mittels Zentrifugalkraft (10 Minuten bei 110×g) durch eine Porex®-Filter-Einheit passagiert, welche eine Höhe von 2,3 mm und eine Porengröße von 16-22 µm aufweist. Die Differenz zwischen der Plättchenanzahl vor und nach Filterpassage der Probe wird bestimmt und der Retention Index (RI %) wird berechnet. Eine verminderte Rückhaltung von Plättchen im Filter zeigt einen Plättchenfunktionsverlust an. Eine erhöhte Rückhaltung von Plättchen im Filter zeigt eine erhöhte Plättchenaktivität an.

Nachteilig bei den beiden letztgenannten Verfahren ist, dass neben der eigentlichen Testdurchführung in jeder Probe zweimal die Plättchenanzahl bestimmt werden muss. Dazu sind erstens besondere Analysegeräte erforderlich und zweitens muss jede Probe mehrfach prozessiert werden.

Diverse im Markt befindliche Geräte zur automatischen Gerinnungsdiagnostik (Gerinnungsanalyzer) umfassen eine Zentrifugationseinheit. Diese besteht üblicherweise aus einem Küvettenrotor, an dem eine Spektrophotometereinheit so angeordnet ist, dass die Proben während der Rotation des Küvettenrotors photometrisch gemessen werden können. Es ist daher besonders wünschenswert ein Verfahren zur Thrombozytendiagnostik bereit zu stellen, das auf den vorhandenen Geräten, die eine Zentrifuagtionseinheit aufweisen, durchführbar wäre.

Der vorliegenden Erfindung lag daher die Aufgabe zugrunde, eine Vorrichtung und ein Verfahren zur Bestimmung der Thrombozytenfunktion bereit zu stellen, die/das eine zuverlässige, einfache und schnelle Thrombozytenfunktionsbestimmung auf einem Zentrifugalanalyzer ermöglicht.

Diese Aufgabe wird durch die technischen Merkmale der unabhängigen Ansprüche gelöst. Die abhängigen Ansprüche geben weitere Ausführungsformen der Erfindung an.

Die vorliegende Erfindung betrifft eine Messzelle zur Bestimmung der Thrombozytenfunktion, wobei die Messzelle folgende Komponenten umfasst:
a) eine erste Kammer zur Aufnahme einer thrombozytenhaltigen Flüssigprobe,
b) eine zweite Kammer, die die Flüssigprobe aus der ersten Kammer auffängt, sofern eine Zentrifugalkraft auf die Messzelle wirkt, und
c) ein poröses Trennelement, das die erste und zweite Kammer voneinander abtrennt,
und wobei das Trennelement mindestens eine die Thrombozytenaktivität beeinflussende, lösliche Substanz enthält.

Die Begriffe "Thrombozyten" und "Plättchen" werden synonym verwendet.

Unter dem Begriff "thrombozytenhaltige Flüssigprobe" ist eine flüssige Probe zu verstehen, die humane oder tierische Thrombozyten enthält, insbesondere Vollblut, plättchenreiches Plasma (PRP) oder andere Plättchenpräparationen. Bevorzugterweise handelt es sich bei der Vollblutprobe um frisch abgenommenes, venöses, antikoaguliertes menschliches oder tierisches Blut. Bevorzugterweise wird das Vollblut durch Zugabe eines Antikoagulanzes antikoaguliert. Zur Verwendung als Antikoagulanz geeignet sind gepufferte, Calcium-bindende Citratlösungen, wie z.B. 3,2 oder 3,8 %ige gepufferte Natrimcitratlösungen, EDTA, Heparin sowie natürliche oder synthetische direkte Thrombininhibitoren, wie z.B. Hirudin, PPACK (D-Phe-Pro-Arg-Chloromethylketon, HCl), Argatroban und Melagatran, oder natürliche oder synthetische direkte Faktor Xa-Inhibitoren, wie z.B. Antistasin, Tick Anticoagulant Peptide, Yagin, Draculin, GGACK (H-Glu-Glu-Arg-Chloromethylketon), Diamidino-Faktor Xa-Inhibitoren und Mono-Benzamidin Faktor Xa-Inhibitoren.

Unter dem Begriff "poröses Trennelement" ist ein Trennelement zu verstehen, das die erste und zweite Kammer voneinander vollständig abtrennt und das aus einem Material besteht, das den Durchtritt einzelner Blutzellen erlaubt, den Durchtritt von Zellaggregaten, insbesondere von Thrombozytenaggregaten aktivierter Thrombozyten jedoch verhindert. Dazu weist das Material vorzugsweise eine Porengröße von 2 - 20 µm, besonders bevorzugt von 5 µm auf. Darüberhinaus ist das Trennelement unversehrt, d.h. es ist frei von Perforationen, Einschnitten oder Aperturen jeglicher Art.

Das poröse Trennelement enthält mindestens eine die Thrombozytenaktivität beeinflussende Substanz, die, wenn eine thrombozytenhaltige Flüssigprobe mit dem Trennelement in Kontakt gebracht wird, in der Flüssigprobe löslich ist.

Bevorzugterweise ist das Trennelement in Form einer Membran ausgestaltet. Das bevorzugte Material ist flüssigkeitsabsorbierend, so dass die die Thrombozytenaktivität beeinflussenden Substanzen in gelöster Form aufgebracht werden können. Besonders bevorzugte Materialien sind Celluloseester, Keramik, Nylon, Polypropylen, Polyethersulfon und Polyvinylidenfluorid (PVDF). Bevorzugterweise wird das mit den gewünschten Substanzen benetzte oder durchtränkte poröse Trennelement getrocknet. Durch den Kontakt der Flüssigprobe mit dem Trennelement werden die Substanzen aus oder von dem Trennelement gelöst und vermischen sich mit der thrombozytenhaltigen Probe.

Der Begriff "die Thrombozytenaktivität beeinflussende Substanz" umfasst Substanzen, die die Aggregation von Thrombozyten zu induzieren oder zu inhibieren vermögen.

In einer Ausführungsform enthält das poröse Trennelement mindestens einen Thrombozytenaktivator, bevorzugt aus der Gruppe ADP (Adenosin-5'-diphosphat), 2-MeSADP (2-Methylthioadenosin-5'-diphosphat), Collagen, Epinephrin, Ristocetin, Thrombin, TRAP (Thrombin-Receptor-Activating-Protein), Arachidonsäure, U46619 ((Z)-7-[(1S,4R,5R,6S)-5-[(E,3S)-3-hydroxyoct-1-enyl]-3-oxabicyclo[2.2.1]heptan-6-yl]hept-5-enolsäure), PMA (Phorbol 12-myristat 13-acetat) und Serotonin.

In einer anderen Ausführungsform enthält das poröse Trennelement mindestens einen Thrombozyteninhibitor, bevorzugt aus der Gruppe Prostaglandin E1 (PGE 1), Prostaglandin I2, Forskolin, Iloprost und Cicaprost.

In anderen Ausführungsformen können ein oder mehrere Substanzen aus der Gruppe der Thrombozytenaktivatoren und der Gruppe der Thrombozyteninhibitoren in dem Trennelement enthalten sein. So eignet sich z.B. die Kombination von ADP und PGE 1 besonders zur Bestimmung der Thrombozytenaktivität in Proben, die von Patienten stammen, die mit einem antithrombotischen Therapeutikum aus der Gruppe der P2Y(12)-Antagonisten, wie z.B. Clopidogrel oder Ticlopidin, behandelt werden. Der Fachmann weiß, welche Substanzen oder welche Substanzkombinationen er verwenden kann, um die Thrombozytenaktivität zu bestimmen.

Bevorzugterweise handelt es sich bei der erfindungsgemäßen Messzelle um einen einteiligen, bevorzugt zylindrischen oder konischen Hohlkörper, in den ein poröses Trennelement passgenau eingesetzt ist, so dass das Trennelement die erste und zweite Kammer vollständig, d.h. über den gesamten Durchmesser des Hohlkörpers, voneinander abtrennt. Bevorzugterweise handelt es sich bei dem einteiligen Hohlkörper um ein Röhrchen mit einem Innendurchmesser von etwa 100 µm bis 1 cm. Die erste Kammer dient der Aufnahme einer thrombozytenhaltigen Flüssigprobe und weist dazu eine Öffnung auf. Die zweite Kammer dient dem Auffangen der Flüssigprobe aus der ersten Kammer, die durch das Trennelement tritt, sofern eine Zentrifugalkraft auf die Messzelle wirkt.

Alternativ kann es sich bei der erfindungsgemäßen Messzelle um ein zweiteiliges Reaktionsgefäß handeln, welches aus einem ersten einseitig offenen Hohlkörper besteht, welcher die zweite Kammer bildet und einem zweiten zweiseitig offenen Hohlkörper, welcher die erste Kammer bildet. Die beiden Hohlkörper-Bauteile sind so verbunden, dass eine Öffnung des zweiten Bauteils auf der einen Öffnung des ersten Bauteils angeordnet ist. Das poröse Trennelement wird entweder genau zwischen den beiden Bauteilen angebracht oder in der einen Öffnung des ersten Bauteils oder in der dem ersten Bauteil zugewandten Öffnung des zweiten Bauteils.

Das einteilige Reaktionsgefäß bzw. die Bauteile des mehrteiligen Reaktionsgefäßes bestehen bevorzugterweise aus einem transparenten Material, bevorzugt aus Kunststoff oder aus Glas.

Eine bevorzugte Ausführungsform der erfindungsgemäßen Messzelle weist im Inneren der zweiten Kammer, die von dem porösen Trennelement aus gesehen entlang ihrer Längsachse eine proximale Hälfte und eine distale Hälfte aufweist, ein Mittel auf, das den Rauminhalt der zweiten Kammer in einen ersten Teilbereich und einen zweiten Teilbereich aufteilt. Der erste Teilbereich dient dem Auffangen und der Weiterleitung der Probenflüssigkeit, die durch das Trennelement hindurchtritt. Die Weiterleitung der Probenflüssigkeit erfolgt von der proximalen in die distale Hälfte der zweiten Kammer. Der zweite Teilbereich dient der Messung der Flüssigkeitsmenge der Probenflüssigkeit, die in der zweiten Kammer aufgefangen wird. Der erste und der zweite Teilbereich stehen in der distalen Hälfte der zweiten Kammer miteinander in Verbindung, so dass die aufgefangene und weitergeleitete Probenflüssigkeit vom ersten Teilbereich in den zweiten Teilbereich übertreten kann. Die Aufteilung des Rauminhalts der zweiten Kammer in einen ersten Teilbereich (Auffangbereich) und einen zweiten Teilbereich (Messbereich) hat den Vorteil, dass die Probenflüssigkeit, die das Trennelement während der Zentrifugation passiert, nicht unkontrolliert die gesamte zweite Kammer benetzt, was zu Störung bei der Messung der Füllhöhe oder eines anderen Parameters in der zweiten Messkammer führen könnte. Die Aufteilung des Rauminhalts der zweiten Kammer in einen ersten Teilbereich (Auffangbereich) und einen zweiten Teilbereich (Messbereich) stellt sicher, dass die Probenflüssigkeit aufgefangen, gesammelt und gezielt in den distalen Bereich der zweiten Kammer weitergeleitet wird, wodurch sichergestellt wird, dass sich die Probenflüssigkeit im zweiten Teilbereich (Messbereich) von unten (distal) nach oben (proximal) in der Messzellenspitze sammelt.

Das Mittel zum Aufteilen des Rauminhalts der zweiten Kammer in einen ersten und einen zweiten Teilbereich kann verschiedenartig ausgestaltet sein.

In einer einfachen Ausgestaltungsform kann das Mittel zum Aufteilen des Rauminhalts der zweiten Kammer in einen ersten und einen zweiten Teilbereich eine relativ zum porösen Trennelement geneigte Ebene aufweisen oder lediglich aus einer durchgehenden Rampe mit einer geneigten Ebene bestehen, die von der proximalen in die distale Hälfte der zweiten Kammer reicht.

In einer anderen Ausgestaltungsform weist das Mittel zum Aufteilen des Rauminhalts der zweiten Kammer in einen ersten und einen zweiten Teilbereich einen ersten Abschnitt auf, der eine relativ zum porösen Trennelement geneigte Ebene aufweist, und einen auf den ersten Teilabschnitt folgenden zweiten Teilabschnitt, der im wesentlichen parallel zu einer den Rauminhalt der zweiten Kammer begrenzenden Wand verläuft und mit dieser eine röhrenförmige Struktur bildet, die bis in die distale Hälfte der zweiten Kammer reicht. Diese Ausgestaltungsform hat den Vorteil, dass der erste Teilbereich der zweiten Kammer (Auffangbereich) auf ein Minimum des Rauminhalts der zweiten Kammer reduziert wird, so dass ein möglichst großer Rauminhalt der zweiten Kammer für den zweiten Teilbereich (Messbereich) zur Verfügung steht. Dies hat zum Beispiel den Vorteil, dass für etwaige Messvorrichtungen, die an der Messzelle angeordnet werden, ein Maxiumum an verfügbarer Fläche zur Verfügung steht.

In einer anderen Ausgestaltungsform ist das Mittel zum Aufteilen des Rauminhalts der zweiten Kammer in einen ersten und einen zweiten Teilbereich trichterförmig ausgestaltet, und der rohrförmige Bereich des trichterförmigen Mittels reicht bis in die distale Hälfte der zweiten Kammer.

Eine Ausführungsform einer Messzelle, die ein Mittel zum Aufteilen des Rauminhalts der zweiten Kammer in einen ersten und einen zweiten Teilbereich aufweist, kann so ausgestaltet sein, dass der zweite Teilbereich der zweiten Kammer (Messbereich) Mittel zur elektrischen Messung des Füllstands aufweist, bevorzugterweise mehrere Elektrodenpaare.

Eine andere Ausführungsform einer erfindungsgemäßen Messzelle weist ein Mittel zum Aufteilen des Rauminhalts der zweiten Kammer in einen ersten, einen zweiten und einen dritten Teilbereich auf. Der erste Teilbereich (Auffangbereich) dient dem Auffangen und der Weiterleitung der Probenflüssigkeit, die durch das Trennelement hindurchtritt, entlang der Längsachse der zweiten Kammer von der proximalen in die distale Hälfte. Der erste Teilbereich steht in der distalen Hälfte der zweiten Kammer mit dem zweiten Teilbereich in Verbindung, so dass die aufgefangene und weitergeleitete Probenflüssigkeit vom ersten Teilbereich in den zweiten Teilbereich übertreten kann. Der zweite Teilbereich (Messbereich) dient dem Messen der Flussgeschwindigkeit der Probenflüssigkeit, die in der zweiten Kammer aufgefangen wird. Der zweite Teilbereich (Messbereich)hat die Form einer Kapillare, die von der distalen Hälfte in Richtung der proximalen Hälfte der zweiten Kammer aufsteigt und die an ihrem Ende eine Öffnung aufweist, die eine Verbindung zu dem dritten Teilbereich herstellt. Der dritte Teilbereich (Überlaufbereich) dient als Überlauf für die Probenflüssigkeit aus dem zweiten Teilbereich. Diese Ausführungsform einer Messzelle ist besonders für die Bestimmung der Thrombozytenfunktion anhand der Messung der Flussgeschwindigkeit der Probenflüssigkeit geeignet.

Ein weiterer Gegenstand der vorliegenden Erfindung betrifft eine Vorrichtung für einen Zentrifugalanalysator, die mindestens zwei erfindungsgemäße Messzellen aufweist. Eine solche Vorrichtung kann je nach Ausgestaltung bis zu 100 Messzellen aufweisen. Der Vorteil einer solchen Vorrichtung besteht darin, dass mehrere Thrombozytenfunktionsbestimmungen simultan durchgeführt werden können. Dabei können entweder mehrere Aliquots einer Probe oder Aliquots verschiedener Proben gleichzeitig bestimmt werden. Die mehreren Messzellen können entweder in Bezug auf ihre bauliche Form oder in Bezug auf die Beschichtung der Trennelemente mit die Thrombozytenaktivität beeinflussenden Substanzen gleichartig oder verschiedenartig sein.

In einer Ausführungsform einer Vorrichtung für einen Zentrifugalanalysator, die mindestens zwei erfindungsgemäße Messzellen aufweist, handelt es sich um eine Scheibe, auf der die Messzellen kreisbogenförmig und radial angeordnet sind. Eine solche Vorrichtung wird im Folgenden auch als Messzellenrotor bezeichnet.

In einer besonderen Ausführungsform eines Messzellenrotors weisen die Messzellen entlang ihrer Längsachse keine gerade, sondern eine gebogene Form auf. Dies hat den Vorteil, dass in den Messzellen konstante Flussbedingungen währen der Rotation des Messzellenrotors herrschen. Bevorzugterweise hat zumindest der Bereich einer Messzelle, der das Trennelement enthält, einen geringeren Durchmesser als die übrigen Bereiche der Messzelle. Bevorzugterweise hat dieser Bereich einen Durchmesser von etwa 50 bis 500 µm.

Ein erfindungsgemäßer Messzellenrotor kann beispielsweise aus lichtdurchlässigem Kunststoff bestehen und/oder aus einem Oberteil und einem Unterteil zusammengesetzt sein. Die im zusammengesetzten Messzellenrotor einander zugewandten Seiten des Ober- und Unterteils können Aussparungen und/oder Erhebungen aufweisen, die dann die gewünschten Messzellenformen ausbilden. Das Oberteil enthält für jede Messzelle ein Pipettierloch, durch welches das Probenmaterial in die erste Kammer einer Messzelle eingeführt werden kann. Das poröse Trennelement kann, bevor das Ober- und Unterteil zusammengefügt werden, entweder als individuelle Einheit separat in jede Messzelle eingesetzt werden oder in Form eines durchgehenden Streifens aus geeignetem porösen Material, der konzentrisch zwischen dem Ober- und Unterteil angeordnet wird und so alle Messzellen des Rotors durchläuft. Die Beschichtung des porösen Materials mit einer oder mehreren die Thrombozytenaktivität beeinflussenden Substanzen kann entweder vor Einsetzen der separaten Trennelemente oder des durchgehenden Streifens erfolgen oder in situ, d.h. wenn die Trennelemente oder der Streifen bereits mit dem Ober-oder Unterteil verbunden wurden.

Ein Messzellenrotor, der zumindest teilweise aus einem transparenten Material besteht, ermöglicht die Messung der Flüssigkeitsmenge, die in der zweiten Kammer aufgefangen wird oder die Messung der Flussgeschwindigkeit der Probenflüssigkeit durch das Trennelement mittels photometrischer Verfahren. Bevorzugterweise sind dazu oberhalb des Messzellenrotors eine oder mehrere mehrere Lichtquellen, bevorzugterweise lichtemittierende Dioden (LED's), und unterhalb des Messzellenrotors jeweils dazugehörige Lichtdetektoren angeordnet, oder umgekehrt, so dass das Licht senkrecht zur Rotationsebene durch die Messbereiche der Messzellen gestrahlt werden kann.

Ein weiterer Gegenstand der vorliegenden Erfindung ist ein Verfahren zur Bestimmung der Thrombozytenfunktion, bei dem eine erfindungsgemäße Messzelle oder eine Vorrichtung, die mindestens zwei erfindungsgemäße Messzellen umfasst, verwendet wird. Das Verfahren umfasst mindestens folgende Schritte:
i. Befüllen der ersten Kammer einer erfindungsgemäßen Messzelle mit einer thrombozytenhaltigen Flüssigprobe,
ii. Ausübung einer Zentrifugalkraft auf die Messzelle mit der Flüssigprobe,
iii. Messen der Flüssigkeitsmenge, die in der zweiten Kammer aufgefangen wird oder Messen der Flussgeschwindigkeit der Probenflüssigkeit durch das Trennelement.

Die Flüssigkeitsmenge (oder das Flüssigkeitsvolumen), die infolge der Einwirkung der Zentrifugalkraft von der ersten Kammer durch das Trennelement tritt und in der zweiten Kammer aufgefangen wird, korreliert invers mit der Thrombozytenfunktion.

Auch die Flussgeschwindigkeit der Probenflüssigkeit, die durch das Trennelement tritt und in der zweiten Kammer aufgefangen wird, korreliert invers mit der Thrombozytenfunktion.

Je weniger Thrombozyten aufgrund einer eingeschränkten Thrombozytenfunktion aggregieren und die Poren des Trennelements verstopfen, desto mehr Probenflüssigkeit kann in einem gegebenen Zeitabschnitt durch das Trennelement hindurchtreten und desto größer ist die Flussgeschwindigkeit bzw. die Durchflussrate und damit die Flüssigkeitsmenge, die in der zweiten Kammer aufgefangen wird.

Je mehr Thrombozyten aufgrund einer verstärkten Thrombozytenfunktion aggregieren und die Poren des Trennelements verstopfen, desto weniger Probenflüssigkeit kann in einem gegebenen Zeitabschnitt durch das Trennelement hindurchtreten und desto geringer ist die Flussgeschwindigkeit bzw. die Durchflussrate und damit die Flüssigkeitsmenge, die in der zweiten Kammer aufgefangen wird.

Vorzugsweise liegt die Zentrifugalkraft, die erfindungsgemäß auf die Messzelle ausgeübt wird, in einem Bereich von 50 - 2.000 × g. Die Zentrifugalkraft kann mit Hilfe einer herkömmlichen Zentrifugationseinheit ausgeübt werden.

Das Messen der Flüssigkeitsmenge, die durch das Trennelement tritt und in der zweiten Kammer aufgefangen wird, kann auf unterschiedliche Art und Weise erfolgen.

In einer ersten Ausführungsform wird die Flüssigkeitsmenge, die in der zweiten Kammer aufgefangen wird, photometrisch bestimmt. Dazu besteht zumindest ein Teilabschnitt der zweiten Kammer der erfindungsgemäßen Messzelle aus einem lichtdurchlässigen Material. Ferner sind entlang der Längsachse der zweiten Kammer und senkrecht zur Rotationsebene der erfindungsgemäßen Messzelle oder des erfindungsgemäßen Messzellenrotors mehrere Lichtquellen, bevorzugterweise lichtemittierende Dioden (LED's), und jeweils dazugehörige Lichtdetektoren angeordnet. Während der Messung senden die Lichtquellen Licht aus, dessen Intensität I durch den jeweils einer Lichtquelle zugeordneten Detektor gemessen wird. Befindet sich zwischen Lichtquelle und Detektor Probenflüssigkeit, wird die Intensität des Lichtes vermindert. Die Extinktion E=-log (Iₜ/I₀) (Iₜ = Lichtintensität zum Zeitpunkt t, I₀ = Lichtintensität zum Zeitpunkt 0) ist proportional zur Menge des absorbierenden Materials zwischen Lichtquelle und Detektor. Die relative Positionierung der Lichtquellen in Bezug auf die Längsachse der zweiten Kammer ergibt eine Aussage auf das durch die Trennschicht geflossene Probenvolumen. Die Anzahl der Lichtquellen/Detektor-Paare gibt die Diskriminierung unterschiedlicher Volumina vor. Im einfachsten Fall (n=3) kann nur eine digitale Aussage über normal oder pathologisch gefällt werden, bei n=6 gibt es 5 mögliche Volumendiskriminierungen. Es wird bestimmt, ob die Extinktion an einem gegebenen Ort größer als ein vorgegebener Schwellenwert (Probenflüssigkeit im Strahlengang) oder niedriger als dieser Schwellenwert (Luft im Strahlengang) ist. Zwischen dem Abstand eines Detektors vom Boden des Gefäßes und der Flüssigkeitsmenge im Gefäß besteht ein einfacher Zusammenhang. Befindet sich ein Detektor in einem Abstand h vom Boden eines zylindrischen Gefäßes mit dem Radius r, so bedeutet eine hohe Extinktion eine Mindestflüssigkeitsmenge im Gefäß von r²πh. Die Messung der Extinktion im Zeitverlauf ermöglicht eine zeitabhängige Bestimmung der Flüssigkeitsmenge: V = V(t). Durch mathematische Ableitung der Gesamtflüssigkeitsmenge nach der Zeit (d/dt V(t)) ergibt sich eine Bestimmung der zeitabhängigen Durchflussgeschwindigkeit.

In einer zweiten Ausführungsform wird die Flüssigkeitsmenge, die in der zweiten Kammer aufgefangen wird, elektrisch bestimmt. Dazu sind an der zweiten Testkammer mehrere Elektrodenpaare unterschiedlicher Länge zur Füllstandserkennung angeordnet. Je größer das Flüssigkeitsvolumen in der zweiten Kammer ist, desto mehr Elektroden haben Kontakt mit der Probenflüssigkeit. Zwischen dem Abstand einer Elektrode vom Boden des Gefäßes und der Flüssigkeitsmenge im Gefäß besteht ein einfacher Zusammenhang. Die Anzahl der Elektroden gibt die Diskriminierung unterschiedlicher Volumina vor. Im einfachsten Fall (n=3) kann nur eine digitale Aussage über normal oder pathologisch gefällt werden, bei n=6 gibt es 5 mögliche Volumendiskriminierungen. Im einfachsten Fall wird über die Leitfähigkeit gemessen (Ohmsche Messung). Befindet sich Luft zwischen den Elektroden, zwischen denen eine Arbeitsspannung anliegt, fließt kein Strom, da Luft ein elektrischer Isolator ist. Füllt sich die Kammer jedoch mit Probenflüssigkeit, kann sie den jeweiligen Stromkreis zwischen Elektroden schließen, da Blut oder Plasma durch gelöste Salze elektrisch leitend sind.

In einer dritten Ausführungsform wird die Flussgeschwindigkeit mittels Laser-Doppler-Anemometrie (LDA) bestimmt. Dazu besteht zumindest ein Teilabschnitt der zweiten Kammer der erfindungsgemäßen Messzelle aus einem lichtdurchlässigen Material. Ein Laserstrahl wird in zwei Strahlen aufgeteilt, die so ausgerichtet werden, dass sie sich in einem Bereich der zweiten Kammer kreuzen. Am Messpunkt, wo sich die Strahlen kreuzen, entsteht ein Interferenzmuster. Ein Detektor misst die beiden Streuwellen, die von der strömenden Probenflüssigkeit erzeugt werden. Das Messsignal ist eine Überlagerung der beiden Streuwellen, wodurch eine durch den Dopplereffekt hervorgerufene Schwebung entsteht, deren Frequenz (Dopplerfrequenz) proportional zur Geschwindigkeit der fließenden Probenflüssigkeit ist.

Die Messung der Flüssigkeitsmenge, die in der zweiten Kammer aufgefangen wird, kann kontinuierlich über einen gegebenen Zeitabschnitt erfolgen. Dazu wird der zeitabhängige Fluss durch dass Trennelement gemessen.

Alternativ kann die Messung der Flüssigkeitsmenge, die in der zweiten Kammer aufgefangen wird, einmalig zu einem gegebenen Zeitpunkt erfolgen. Beispielsweise kann die Messung über eine Endpunktbestimmung erfolgen. Dazu wird die Gesamtflüssigkeitsmenge bestimmt, die zu einem bestimmten Zeitpunkt das Trennelement passiert hat.

Die Bestimmung der Thrombozytenfunktion in einer unbekannten Probe erfolgt bevorzugterweise durch den Vergleich des Messergebnisses der Probe mit dem Messergebnis für ein oder mehrere Kontrollen mit einer bekannten Thrombozytenaktivität. Bevorzugterweise bestehen die Kontrollen/Kalibratoren aus einem Kollektiv von Blut-/Plasmaspenden gesunder Personen. Aus den Messergebnissen, die für die Proben dieses Kollektivs bestimmt wurden, wird vorzugsweise der Median bestimmt und das Messergebnis einer unbekannten Probe wird dazu in Relation gesetzt.

Da die Menge der Probenflüssigkeit, die das Trennelement durchtritt, invers proportional zur Thrombozytenfunktion ist, eignet sich inbesondere der Kehrwert der in der zweiten Kammer aufgefangenen Flüssigkeitsmenge V (1 / V) als Maß für die Thrombozytenfunktion. Liegt der Kehrwert 1 / V einer unbekannten Probe unterhalb eines zuvor ermittelten Mediangrenzwertes, handelt es sich um eine Probe mit eingeschränkter Thrombozytenfunktion und einem Blutungsrisiko. Liegt der Kehrwert 1 / V einer unbekannten Probe oberhalb eines zuvor ermittelten Mediangrenzwertes, handelt es sich um eine Probe mit verstärkter Thrombozytenfunktion und einem Thromboserisiko.

### FIGURENBESCHREIBUNG

Die vorliegende Erfindung wird durch die gezeigten und im Folgenden diskutierten Figuren genauer erläutert. Dabei ist zu beachten, dass die Figuren nur beschreibenden Charakter haben und nicht dazu gedacht sind, die Erfindung in irgendeiner Form einzuschränken.
**Figur 1**

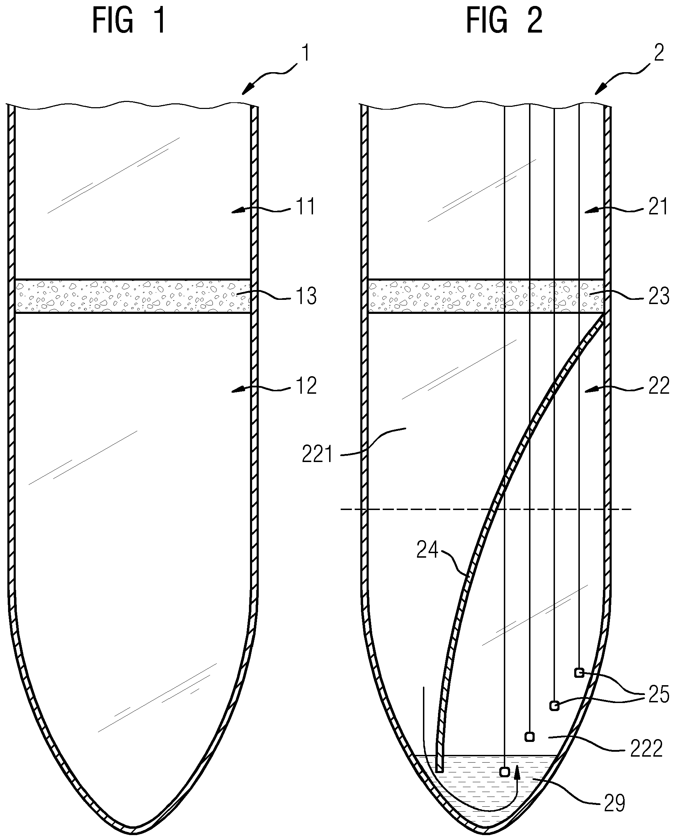

   Fig. 1 zeigt eine Ausführungsform einer erfindungsgemäßen Messzelle (1). Die Messzelle (1) umfasst eine erste Kammer (11) zur Aufnahme einer thrombozytenhaltigen Flüssigprobe und eine zweite Kammer (12), die die Flüssigprobe aus der ersten Kammer auffängt, sofern eine Zentrifugalkraft auf die Messzelle wirkt. Die Messzelle umfasst ferner ein poröses Trennelement (13), das die erste und zweite Kammer über den gesamten Durchmesser des Hohlkörpers, voneinander abtrennt. Das Trennelement (13) enthält mindestens eine die Thrombozytenaktivität beeinflussende, lösliche Substanz.
**Figur 2**
   Fig. 2 zeigt eine andere Ausführungsform einer erfindungsgemäßen Messzelle (2). Die Messzelle (2) umfasst eine erste Kammer (21) zur Aufnahme einer thrombozytenhaltigen Flüssigprobe und eine zweite Kammer (22), die die Flüssigprobe aus der ersten Kammer auffängt, sofern eine Zentrifugalkraft auf die Messzelle wirkt. Die Messzelle umfasst ferner ein poröses Trennelement (23), das die erste und zweite Kammer über den gesamten Durchmesser des Hohlkörpers, voneinander abtrennt. Das Trennelement (23) enthält mindestens eine die Thrombozytenaktivität beeinflussende, lösliche Substanz.
   Darüberhinaus verfügt die hier gezeigte Messzelle (2) in der zweiten Kammer (22) über ein Mittel (24), in Form eines rampenförmigen Elements, zum Aufteilen des Rauminhalts der zweiten Kammer in einen ersten Teilbereich (221) und einen zweiten Teilbereich (222).
   Der erste Teilbereich (221) dient dem Auffangen und der Weiterleitung der Probenflüssigkeit, die durch das Trennelement hindurchtritt. Der zweite Teilbereich (222) dient dem Messen der Flüssigkeitsmenge der Probenflüssigkeit (29), die in der zweiten Kammer (22) aufgefangen wird. Der erste und zweite Teilbereich stehen in der distalen Hälfte der zweiten Kammer (d.h. unterhalb der gestrichelten Linie) miteinander in Verbindung, so dass die aufgefangene und weitergeleitete Probenflüssigkeit erst in Nähe der Messzellenspitze vom ersten Teilbereich in den zweiten Teilbereich übertreten kann. Dies hat den Vorteil, dass die Probenflüssigkeit, die das Trennelement (23) während der Zentrifugation passiert, nicht unkontrolliert die gesamte zweite Kammer (24) benetzt, sondern dass die Probenflüssigkeit aufgefangen, gesammelt und gezielt in den distalen Bereich der zweiten Kammer weitergeleitet wird. Die so weitergeleitete Probenflüssigkeit (29) sammelt sich in der zweiten Kammer (22), insbesondere im zweiten Teilbereich (222) der zweiten Kammer (22) von unten (distal) nach oben (proximal) in der Messzellenspitze.
   Darüberhinaus verfügt die hier gezeigte Messzelle (2) über Elektroden (25) unterschiedlicher Länge zur Füllstandserkennung. Je höher der Flüssigkeitspegel in der zweiten Kammer (22), insbesondere im zweiten Teilbereich (222) der zweiten Kammer (22), steigt, desto mehr Elektroden haben Kontakt mit der Probenflüssigkeit.
**Figur 3**

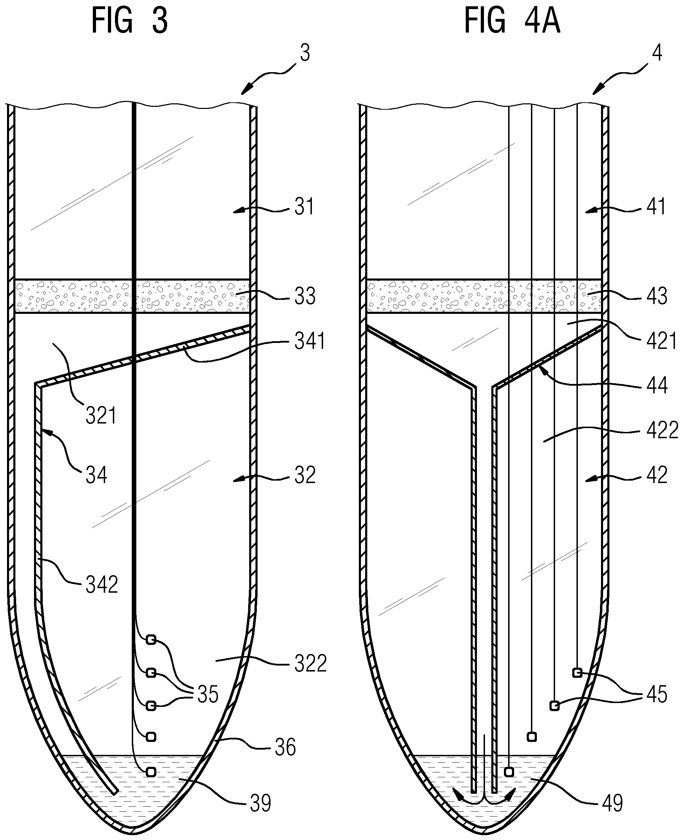

   Fig. 3 zeigt eine weitere Ausführungsform einer erfindungsgemäßen Messzelle (3). Die Messzelle (3) umfasst eine erste Kammer (31) zur Aufnahme einer thrombozytenhaltigen Flüssigprobe und eine zweite Kammer (32), die die Flüssigprobe aus der ersten Kammer auffängt, sofern eine Zentrifugalkraft auf die Messzelle wirkt. Die Messzelle umfasst ferner ein poröses Trennelement (33), das die erste und zweite Kammer über den gesamten Durchmesser des Hohlkörpers, voneinander abtrennt. Das Trennelement (33) enthält mindestens eine die Thrombozytenaktivität beeinflussende, lösliche Substanz.
   Darüberhinaus verfügt die hier gezeigte Messzelle (3) in der zweiten Kammer (32) über ein Mittel (34) zum Aufteilen des Rauminhalts der zweiten Kammer in einen ersten Teilbereich (321) und einen zweiten Teilbereich (322).
   Das Mittel (34) zum Aufteilen des Rauminhalts der zweiten Kammer (32) in einen ersten und einen zweiten Teilbereich weist einen ersten Abschnitt (341) auf, der eine relativ zum porösen Trennelement geneigte Ebene aufweist. Das Mittel (34) weist ferner einen auf den ersten Teilabschnitt (341) folgenden zweiten Teilabschnitt (342) auf, der im wesentlichen parallel zu einer den Rauminhalt der zweiten Kammer (32) begrenzenden Wand (36) verläuft und mit dieser eine röhrenförmige Struktur bildet, die bis in die distale Hälfte der zweiten Kammer (32) reicht. Diese Ausgestaltungsform hat den Vorteil, dass der erste Teilbereich (321) der zweiten Kammer, der Auffangbereich, auf ein Minimum des Rauminhalts der zweiten Kammer (32) reduziert wird, so dass ein möglichst großer Rauminhalt der zweiten Kammer (32) für den zweiten Teilbereich (322), den Messbereich, zur Verfügung steht. Dies hat zum Beispiel den Vorteil, dass für etwaige Messvorrichtungen, die an der Messzelle angeordnet werden, ein Maximum an verfügbarer Fläche zur Verfügung steht.
   Ferner verfügt die hier gezeigte Messzelle (3) über Elektroden (35) unterschiedlicher Länge zur Füllstandserkennung. Je höher der Flüssigkeitspegel in der zweiten Kammer (32), insbesondere im zweiten Teilbereich (322) der zweiten Kammer (32), steigt, desto mehr Elektroden haben Kontakt mit der Probenflüssigkeit (39).
**Figur 4**

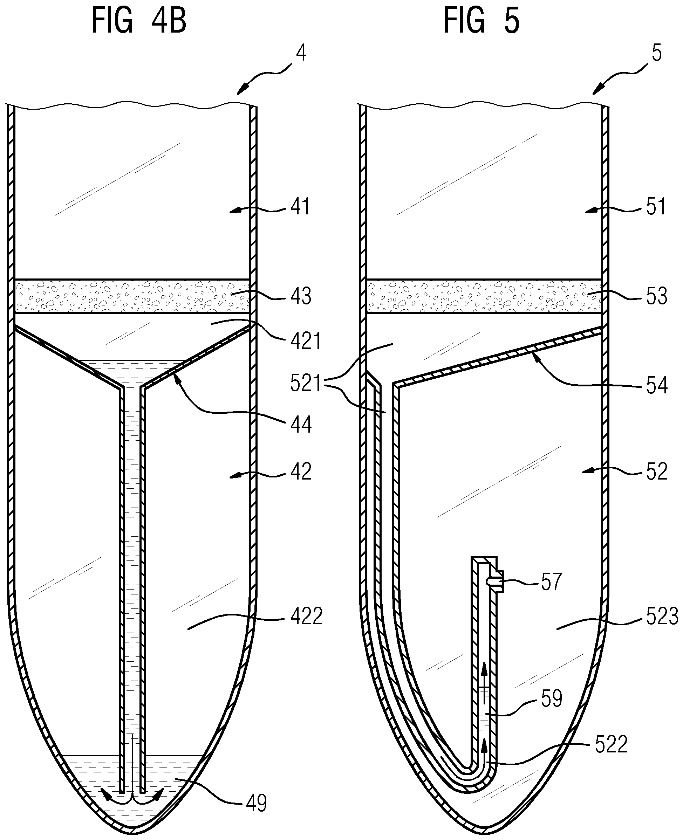

   Fig. 4 zeigt eine weitere Ausführungsform einer erfindungsgemäßen Messzelle (4). Die Messzelle (4) umfasst eine erste Kammer (41) zur Aufnahme einer thrombozytenhaltigen Flüssigprobe und eine zweite Kammer (42), die die Flüssigprobe aus der ersten Kammer auffängt, sofern eine Zentrifugalkraft auf die Messzelle wirkt. Die Messzelle umfasst ferner ein poröses Trennelement (43), das die erste und zweite Kammer über den gesamten Durchmesser des Hohlkörpers, voneinander abtrennt. Das Trennelement (43) enthält mindestens eine die Thrombozytenaktivität beeinflussende, lösliche Substanz.
   Darüberhinaus verfügt die hier gezeigte Messzelle (4) in der zweiten Kammer (42) über ein Mittel (44) zum Aufteilen des Rauminhalts der zweiten Kammer in einen ersten Teilbereich (421) und einen zweiten Teilbereich (422).
   Das Mittel (44) zum Aufteilen des Rauminhalts der zweiten Kammer (42) in einen ersten und einen zweiten Teilbereich ist hier trichterförmig ausgestaltet, und der rohrförmige Bereich des trichterförmigen Mittels reicht bis in die distale Hälfte der zweiten Kammer (42).
   Die in Fig. 4A gezeigte Messzelle (4) verfügt über Elektroden (45) unterschiedlicher Länge zur Füllstandserkennung.
   Bei der in Fig. 4B gezeigten Messzelle (4) bestehen die Wände der Messzelle und zumindest der rohrförmige Bereich des trichterförmigen Mittels (42) aus einem lichtdurchlässigen Material. Diese Ausgestaltungform einer Messzelle eignet sich besonders zur Messung der Flussgeschwindigkeit der Probenflüssigkeit (49) in der zweiten Kammer der Messzelle. Die Flussgeschwindigkeit wird dann in dem rohrförmigen Bereich des trichterförmigen Mittels (44) photometrisch gemessen, mit Hilfe von entlang der Längsachse der Messzelle angeordneten Lichtquellen und Lichtdetektoren (nicht dargestellt). Alternativ kann die Flussgeschwindigkeit in dem rohrförmigen Bereich des trichterförmigen Mittels (44) auch mittels Laser-Doppler-Anemometrie (LDA) gemessen werden.
**Figur 5**
   Fig. 5 zeigt eine weitere Ausführungsform einer erfindungsgemäßen Messzelle (5). Die Messzelle (5) umfasst eine erste Kammer (51) zur Aufnahme einer thrombozytenhaltigen Flüssigprobe und eine zweite Kammer (52), die die Flüssigprobe aus der ersten Kammer auffängt, sofern eine Zentrifugalkraft auf die Messzelle wirkt. Die Messzelle umfasst ferner ein poröses Trennelement (53), das die erste und zweite Kammer über den gesamten Durchmesser des Hohlkörpers, voneinander abtrennt. Das Trennelement (53) enthält mindestens eine die Thrombozytenaktivität beeinflussende, lösliche Substanz.
   Darüberhinaus verfügt die hier gezeigte Messzelle (5) in der zweiten Kammer (52) über ein Mittel (54) zum Aufteilen des Rauminhalts der zweiten Kammer in einen ersten Teilbereich (521), einen zweiten Teilbereich (522) und einen dritten Teilbereich (523). Der erste Teilbereich (521), der Auffangbereich, dient dem Auffangen und der Weiterleitung der Probenflüssigkeit, die durch das Trennelement hindurchtritt, entlang der Längsachse der zweiten Kammer (52) von der proximalen in die distale Hälfte. Der erste Teilbereich (521) steht in der distalen Hälfte der zweiten Kammer (52) mit dem zweiten Teilbereich (522) in Verbindung, so dass die aufgefangene und weitergeleitete Probenflüssigkeit vom ersten Teilbereich (521) in den zweiten Teilbereich (522) übertreten kann. Der zweite Teilbereich (522), der Messbereich, dient dem Messen der Flussgeschwindigkeit der Probenflüssigkeit, die in der zweiten Kammer (52) aufgefangen wird. Der zweite Teilbereich (522) hat hier die Form einer Kapillare, die von der distalen Hälfte in Richtung der proximalen Hälfte der zweiten Kammer aufsteigt und die an ihrem Ende eine Öffnung (57) aufweist, die eine Verbindung zu dem dritten Teilbereich (523) herstellt. Der dritte Teilbereich (523), der Überlaufbereich, dient als Überlauf für die Probenflüssigkeit (59) aus dem zweiten Teilbereich (522). Diese Ausführungsform einer Messzelle ist besonders für die Bestimmung der Thrombozytenfunktion anhand der Messung der Flussgeschwindigkeit der Probenflüssigkeit geeignet.
**Figur 6**

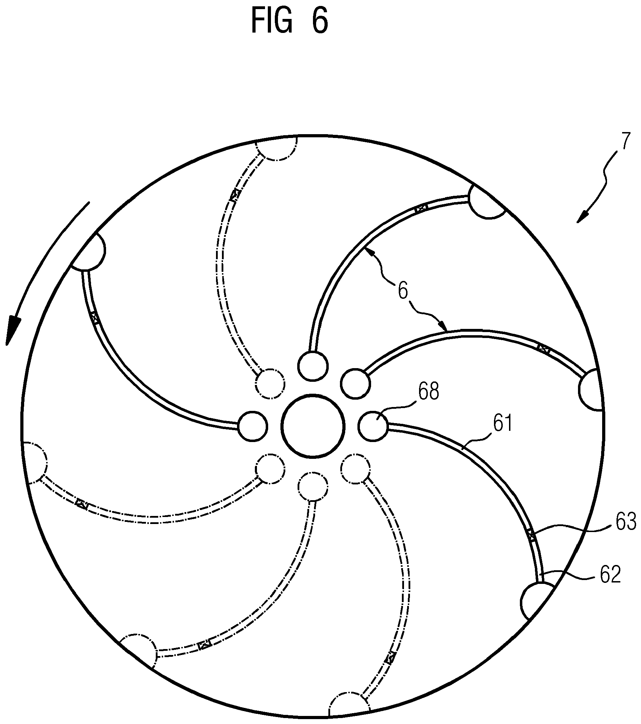

   Fig. 6 zeigt die Aufsicht eines erfindungsgemäßen Messzellenrotors (7) mit mehreren kreisbogenförmig angeordneten Messzellen (6), wobei jede Messzelle (6) eine erste Kammer (61) zur Aufnahme einer thrombozytenhaltigen Flüssigprobe umfasst sowie eine zweite Kammer (62), die die Flüssigprobe aus der ersten Kammer auffängt, sofern eine Zentrifugalkraft auf die Messzelle wirkt. Die Messzelle umfasst ferner ein poröses Trennelement (63), das die erste und zweite Kammer über den gesamten Durchmesser des Hohlkörpers, voneinander abtrennt. Das Trennelement (63) enthält mindestens eine die Thrombozytenaktivität beeinflussende, lösliche Substanz.

Die Messzellen (6) weisen entlang ihrer Längsachse eine gebogene Form auf. Der Bereich einer Messzelle (6), der das Trennelement (63) enthält, hat einen geringeren Durchmesser als die übrigen Bereiche der Messzelle. In der Oberseite des Messzellenrotors (7) befinden sich Pipettierlöcher (68), durch welche das Probenmaterial in die erste Kammer (61) einer Messzelle (6) eingeführt werden kann. Der Pfeil gibt die Rotationsrichtung des Messzellenrotors (7) an.

**BEZUGSZEICHENLISTE**

| | |
|---|---|
| Messzelle | 1, 2, 3, 4, 5, 6 |
| Messzellenrotor | 7 |
| erste Kammer zur Aufnahme einer Probe | 11, 21, 31, 41, 51, 61 |
| zweite Kammer zum Auffangen der Probe aus der ersten Kammer | 12, 22, 32, 42, 52, 62 |
| Trennelement | 13, 23, 33, 43, 53, 63 |
| Mittel zum Aufteilen des Rauminhalts der zweiten Kammer | 24, 34, 44, 54 |
| erster Teilbereich der zweiten Kammer (Auffangbereich) | 221, 321, 421, 521 |
| zweiter Teilbereich der zweiten Kammer (Messbereich) | 222, 322, 422, 522 |
| Elektrode | 25, 35, 45 |
| Probenflüssigkeit | 29, 39, 49, 59 |
| erster Abschnitt eines Mittel zum Aufteilen des Rauminhalts der zweiten Kammer | 341 |
| zweiter Abschnitt eines Mittel zum Aufteilen des Rauminhalts der zweiten Kammer | 342 |
| Wand | 36 |
| dritter Teilbereich der zweiten Kammer (Überlaufbereich) | 523 |
| Öffnung | 57 |
| Pipettierloch | 68 |

Das folgende Ausführungsbeispiel dient der Veranschaulichung des erfindungsgemäßen Verfahrens und ist nicht als Einschränkung zu verstehen.

### BEISPIELE

### BEISPIEL 1: Erfindungsgemäße Bestimmung der Thrombozytenaktivität in einer Vollblutprobe

Erfindungsgemäße Messzellen wurden wie folgt hergestellt: konisch geformte Zentrifugenröhrchen (50 mL-Falcon-Röhrchen aus transparentem Kunststoff, Becton Dickson) wurden auf etwa halber Höhe abgeschnitten. Auf der Öffnung eines abgeschnittenen Falcon-Röhrchens wurde ein Einmalfilteraufsatz mit einer PVDF-Membran (Millipore Millex®-SV, 5 µm Porengröße) angebracht. Erfindungsgemäß wurde die PVDF-Membran mit einer Thrombozytenaktivatormischung enthaltend Kollagen und Epinephrin (jeweils 0,5 mg/mL) vorbehandelt. Dazu wurden 0,8 mL der Mischung über einen Millipore Millex®-SV Einmalfilteraufsatz gegeben und anschließend luftgetrocknet. Zur Kontrolle wurden 0,8 mL Wasser über einen Millipore Millex®-SV Einmalfilteraufsatz gegeben und anschließend luftgetrocknet. Auf dem auf dem abgeschnittenen Falcon-Röhrchen angebrachten Millipore Millex®-SV Einmalfilteraufsatz wurde nun eine Einmalspritze aus Kunststoff (Omnifix®, 5 mL, B. Braun Melsungen AG) ohne Kolben und ohne Injektionsnadel befestigt.

In die erste Kammer der Messzelle, die Einmalspritze, wurden nun 1,5 mL einer normalen Citratblutprobe eingebracht, und die Messzelle wurde in einer Zentrifuge (Rotixa R50, Andreas Hettich GmbH & Co. KG) für 75 Sekunden mit 50 × g bei 22 °C zentrifugiert. Die Flüssigkeitsmenge, die in der zweiten Kammer, dem abgeschnittenen Falcon-Röhrchen aufgefangen wurde, wurde volumetrisch bestimmt.

Wie aus Tabelle 1 ersichtlich ist, bewirkt die Verwendung von mit den Plättchenaktivatoren Kollagen und Epinephrin imprägnierten Trennelementen einen verminderten Durchfluss von Probenflüssigkeit infolge der in der Blutprobe induzierten Plättchenaggregation.

**Tabelle 1**

| **Imprägnierung des Trennelements** | **Durchflussvolumen** |
|---|---|
| Kollagen/Epinephrin | 0,6 mL |
| Wasser | 1,5 mL |

## Patentansprüche

1. Messzelle (1, 2, 3, 4, 5, 6) zur Bestimmung der Thrombozytenfunktion umfassend
a. eine erste Kammer (11, 21, 31, 41, 51, 61) zur Aufnahme einer thrombozytenhaltigen Flüssigprobe,
b. eine zweite Kammer (12, 22, 32, 42, 52, 62), die die Flüssigprobe aus der ersten Kammer auffängt, sofern eine Zentrifugalkraft auf die Messzelle wirkt, und
c. ein poröses Trennelement (13, 23, 33, 43, 53, 63), das die erste und zweite Kammer voneinander abtrennt,
**dadurch gekennzeichnet, dass**
das Trennelement (13, 23, 33, 43, 53, 63) mindestens eine die Thrombozytenaktivität beeinflussende, lösliche Substanz enthält.

2. Messzelle nach Anspruch 1, wobei die mindestens eine die Thrombozytenaktivität beeinflussende, lösliche Substanz ein Thrombozytenaktivator ist, bevorzugt aus der Gruppe ADP, 2-MeSADP, Collagen, Epinephrin, Ristocetin, Thrombin, TRAP, Arachidonsäure, U46619, PMA und Serotonin.

3. Messzelle nach Anspruch 1, wobei die mindestens eine die Thrombozytenaktivität beeinflussende, lösliche Substanz ein Thrombozyteninhibitor ist, bevorzugt aus der Gruppe Prostaglandin E1, Prostaglandin I2, Forskolin, Iloprost und Cicaprost.

4. Messzelle nach einem der vorhergehenden Ansprüche, wobei das Trennelement zusätzlich Calciumchloridionen enthält.

5. Messzelle nach einem der vorhergehenden Ansprüche, wobei das Trennelement (13, 23, 33, 43, 53, 63) eine Porengröße von 2 - 20 µm, bevorzugt von 5 µm aufweist.

6. Messzelle nach einem der vorhergehenden Ansprüche, wobei mindestens ein Teilabschnitt der zweiten Kammer (12, 22, 32, 42, 52, 62) aus einem lichtdurchlässigen Material besteht.

7. Messzelle nach einem der Ansprüche 1 - 6, wobei an der zweiten Kammer (12, 22, 32, 42, 52, 62)mehrere Elektrodenpaare (25, 35, 45) zur elektrischen Füllstandsmessung angeordnet sind.

8. Messzelle (2, 3, 4, 5) nach einem der Ansprüche 1 - 7, wobei die zweite Kammer (22, 32, 42, 52), welche ausgehend von dem porösen Trennelement entlang ihrer Längsachse eine proximale Hälfte und eine distale Hälfte aufweist, im Inneren ein Mittel (24, 34, 44, 54) zum Aufteilen des Rauminhalts der zweiten Kammer in einen ersten Teilbereich (221, 321, 421, 521) und einen zweiten Teilbereich (222, 322, 422, 522) aufweist,
- wobei der erste Teilbereich dem Auffangen und der Weiterleitung der Probenflüssigkeit, die durch das Trennelement hindurchtritt, dient, und
- wobei der zweite Teilbereich dem Messen der Flüssigkeitsmenge der Probenflüssigkeit, die in der zweiten Kammer aufgefangen wird, dient, und
- wobei der erste und zweite Teilbereich in der distalen Hälfte der zweiten Kammer miteinander in Verbindung stehen, so dass die aufgefangene und weitergeleitete Probenflüssigkeit vom ersten Teilbereich in den zweiten Teilbereich übertreten kann.

9. Messzelle (2) nach Anspruch 8, wobei das Mittel (24) zum Aufteilen des Rauminhalts der zweiten Kammer (22) in einen ersten und einen zweiten Teilbereich (221, 222) eine relativ zum porösen Trennelement geneigte Ebene aufweist, die von der proximalen in die distale Hälfte der zweiten Kammer reicht.

10. Messzelle (3) nach Anspruch 8, wobei das Mittel (34) zum Aufteilen des Rauminhalts der zweiten Kammer (32) in einen ersten und einen zweiten Teilbereich (321, 322)
- einen ersten Abschnitt (341) aufweist, der eine relativ zum porösen Trennelement geneigte Ebene aufweist, und
- einen auf den ersten Teilabschnitt folgenden zweiten Teilabschnitt (342) aufweist, der parallel zu einer den Rauminhalt der zweiten Kammer begrenzenden Wand (36) verläuft und mit dieser eine röhrenförmige Struktur bildet, die bis in die distale Hälfte der zweiten Kammer reicht.

11. Messzelle (4) nach Anspruch 8, wobei das Mittel (44) zum Aufteilen des Rauminhalts der zweiten Kammer (42) in einen ersten und einen zweiten Teilbereich (421, 422) trichterförmig ausgestaltet ist und der rohrförmige Bereich des trichterförmigen Mittels bis in die distale Hälfte der zweiten Kammer reicht.

12. Messzelle (2, 3, 4) nach einem der Ansprüche 8 - 11, wobei der zweite Teilbereich (222, 322, 422) der zweiten Kammer, der dem Messen der Flüssigkeitsmenge der Probenflüssigkeit, die in der zweiten Kammer aufgefangen wird, dient, Mittel zur elektrischen Messung des Füllstands aufweist, bevorzugterweise mehrere Elektrodenpaare (25, 35, 45).

13. Messzelle (5) nach einem der Ansprüche 1 - 6, wobei die zweite Kammer (52), welche ausgehend von dem porösen Trennelement entlang ihrer Längsachse eine proximale Hälfte und eine distale Hälfte aufweist, im Inneren ein Mittel (54) zum Aufteilen des Rauminhalts der zweiten Kammer in einen ersten, einen zweiten und einen dritten Teilbereich (521, 522, 523) aufweist,
- wobei der erste Teilbereich (521) dem Auffangen und der Weiterleitung der Probenflüssigkeit, die durch das Trennelement (53) hindurchtritt, dient, und wobei die Weiterleitung der Probenflüssigkeit entlang der Längsachse der zweiten Kammer (52) von der proximalen in die distale Hälfte erfolgt, und
- wobei der zweite Teilbereich (522) dem Messen der Flussgeschwindigkeit der Probenflüssigkeit (59), die in der zweiten Kammer (52) aufgefangen wird, dient, und
- wobei der dritte Teilbereich (523) als Überlauf für die Probenflüssigkeit aus dem zweiten Teilbereich (522) dient, und
- wobei der erste und zweite Teilbereich (521, 522) in der distalen Hälfte der zweiten Kammer miteinander in Verbindung stehen, so dass die aufgefangene und weitergeleitete Probenflüssigkeit vom ersten Teilbereich in den zweiten Teilbereich übertreten kann, und
- wobei der zweite Teilbereich (522) die Form einer Kapillare hat, die von der distalen Hälfte in Richtung der proximalen Hälfte der zweiten Kammer aufsteigt und die an ihrem Ende eine Öffnung (57) aufweist, die eine Verbindung zu dem dritten Teilbereich (523) herstellt.

14. Vorrichtung (7) zur simultanen Bestimmung der Thrombozytenfunktion in mehreren Proben, **dadurch gekennzeichnet, dass** sie mindestens zwei Messzellen (6) gemäß einem der Ansprüche 1 - 13 aufweist.

15. Verfahren zur Bestimmung der Thrombozytenfunktion, das folgende Schritte umfasst:
i. Befüllen der ersten Kammer einer Messzelle gemäß einem der Ansprüche 1-13 mit einer thrombozytenhaltigen Flüssigprobe,
ii. Ausübung einer Zentrifugalkraft auf die Messzelle mit der Flüssigprobe,
iii. Messen der Flüssigkeitsmenge, die in der zweiten Kammer aufgefangen wird oder Messen der Flussgeschwindigkeit der Probenflüssigkeit in der zweiten Kammer der Messzelle,
wobei die aufgefangene Flüssigkeitsmenge und die Flussgeschwindigkeit mit der Thrombozytenfunktion invers korrelieren.

16. Verfahren gemäß Anspruch 15, wobei die Flüssigkeitsmenge, die in der zweiten Kammer der Messzelle aufgefangen wird, photometrisch oder elektrisch bestimmt wird.

17. Verfahren gemäß Anspruch 15, wobei die Flussgeschwindigkeit der Probenflüssigkeit in der zweiten Kammer der Messzelle mittels Laser-Doppler Anemometrie bestimmt wird.

18. Verfahren gemäß einem der Ansprüche 15-17, wobei die Flüssigkeitsmenge, die in der zweiten Kammer der Messzelle aufgefangen wird, oder die Flussgeschwindigkeit kontinuierlich über einen gegebenen Zeitabschnitt gemessen wird.

19. Verfahren gemäß einem der Ansprüche 15-17, wobei die Flüssigkeitsmenge, die in der zweiten Kammer der Messzelle aufgefangen wird, oder die Flussgeschwindigkeit einmalig zu einem gegebenen Zeitpunkt gemessen wird.

20. Verfahren gemäß einem der Ansprüche 15-19, wobei die Flüssigkeitsmenge, die in der zweiten Kammer der Messzelle aufgefangen wird, oder die Flussgeschwindigkeit während der Ausübung der Zentrifugalkraft auf die Messzelle gemessen wird.

21. Verfahren gemäß einem der Ansprüche 15-20, wobei die Zentrifugalkraft, die auf die Messzelle ausgeübt wird, in einem Bereich von 50 - 2.000 × g liegt.

## Claims

1. Measuring cell (1, 2, 3, 4, 5, 6) for determining the platelet function, comprising
a. a first chamber (11, 21, 31, 41, 51, 61) for accommodating a platelet-containing liquid sample,
b. a second chamber (12, 22, 32, 42, 52, 62), which catches the liquid sample from the first chamber provided that a centrifugal force acts on the measuring cell, and
c. a porous divider (13, 23, 33, 43, 53, 63), which separates the first and second chambers from one another,
**characterized in that**
the divider (13, 23, 33, 43, 53, 63) contains at least one soluble substance that influences the platelet activity.

2. Measuring cell according to Claim 1, wherein the at least one soluble substance that influences the platelet activity is a platelet activator, preferably from the following group: ADP, 2-MeSADP, collagen, epinephrine, ristocetin, thrombin, TRAP, arachidonic acid, U46619, PMA and serotonin.

3. Measuring cell according to Claim 1, wherein the at least one soluble substance that influences the platelet activity is a platelet inhibitor, preferably from the following group: prostaglandin E1, prostaglandin 12, forskolin, iloprost and cicaprost.

4. Measuring cell according to one of the preceding claims, wherein the divider additionally contains calcium chloride ions.

5. Measuring cell according to one of the preceding claims, wherein the divider (13, 23, 33, 43, 53, 63) has a pore dimension of 2-20 µm, preferably of 5 µm.

6. Measuring cell according to one of the preceding claims, wherein at least one partial section of the second chamber (12, 22, 32, 42, 52, 62) consists of a light-transmitting material.

7. Measuring cell according to one of Claims 1-6, wherein a plurality of electrode pairs (25, 35, 45) are arranged on the second chamber (12, 22, 32, 42, 52, 62) for electrically measuring the filling level.

8. Measuring cell (2, 3, 4, 5) according to one of Claims 1-7, wherein the second chamber (22, 32, 42, 52), which, starting from the porous divider, has a proximal half and a distal half along the longitudinal axis thereof, has means (24, 34, 44, 54) in the interior for subdividing the space in the second chamber into a first portion (221, 321, 421, 521) and a second portion (222, 322, 422, 522),
- wherein the first portion serves to capture and transport the sample liquid that passes through the divider, and
- wherein the second portion serves to measure the amount of liquid in the sample liquid caught in the second chamber, and
- wherein the first and second portions are interconnected in the distal half of the second chamber such that the caught and transported sample liquid can pass into the second portion from the first portion.

9. Measuring cell (2) according to Claim 8, wherein the means (24) for subdividing the space in the second chamber (22) into a first and a second portion (221, 222) has a plane that is at an angle relative to the porous divider and extends from the proximal into the distal half of the second chamber.

10. Measuring cell (3) according to Claim 8, wherein the means (34) for subdividing the space in the second chamber (32) into a first and a second portion (321, 322)
- has a first section (341), which has a plane that is at an angle relative to the porous divider, and
- has a second partial section (342), which follows the first partial section, runs parallel to a wall (36) delimiting the space in the second chamber and forms a tubular structure with said wall, which tubular structure extends into the distal half of the second chamber.

11. Measuring cell (4) according to Claim 8, wherein the means (44) for subdividing the space in the second chamber (42) into a first and a second portion (421, 422) has a funnel-like shape, and the tubular region of the funnel-like means extends into the distal half of the second chamber.

12. Measuring cell (2, 3, 4) according to one of Claims 8-11, wherein the second portion (222, 322, 422) of the second chamber, which serves to measure the amount of liquid in the sample liquid caught in the second chamber, has means for electrically measuring the filling level, preferably a plurality of electrode pairs (25, 35, 45).

13. Measuring cell (5) according to one of Claims 1-6, wherein the second chamber (52), which, starting from the porous divider, has a proximal half and a distal half along the longitudinal axis thereof, has means (54) in the interior for subdividing the space in the second chamber into a first, a second and a third portion (521, 522, 523),
- wherein the first portion (521) serves to capture and transport the sample liquid that passes through the divider (53) and wherein the sample liquid is transported along the longitudinal axis of the second chamber (52) into the distal from the proximal half, and
- wherein the second portion (522) serves to measure the flow speed of the sample liquid (59) captured in the second chamber (52), and
- wherein the third portion (523) serves as an overflow for the sample liquid from the second portion (522), and
- wherein the first and second portions (521, 522) are interconnected in the distal half of the second chamber such that the sample liquid caught and transported can pass into the second portion from the first portion, and
- wherein the second portion (522) has the form of a capillary, which rises from the distal half in the direction of the proximal half of the second chamber and has an opening (57) at its end, which opening establishes a connection to the third portion (523).

14. Device (7) for simultaneously determining the platelet function in a plurality of samples, **characterized in that** it comprises at least two measuring cells (6) according to one of Claims 1-13.

15. Method for determining the platelet function, which comprises the following steps:
i. filling a platelet-containing liquid sample into the first chamber of a measuring cell according to one of Claims 1-13,
ii. applying a centrifugal force on the measuring cell with the liquid sample,
iii. measuring the amount of liquid caught in the second chamber or measuring the flow speed of the sample liquid in the second chamber of the measuring cell,
wherein the amount of liquid caught and the flow speed are inversely correlated to the platelet function.

16. Method according to Claim 15, wherein the amount of liquid caught in the second chamber of the measuring cell is determined by photometric or electric means.

17. Method according to Claim 15, wherein the flow speed of the sample liquid in the second chamber of the measuring cell is determined by means of laser-Doppler anemometry.

18. Method according to one of Claims 15-17, wherein the amount of liquid caught in the second chamber of the measuring cell or the flow speed is measured continuously over a given time interval.

19. Method according to one of Claims 15-17, wherein the amount of liquid caught in the second chamber of the measuring cell or the flow speed is measured once at a given time.

20. Method according to one of Claims 15-19, wherein the amount of liquid caught in the second chamber of the measuring cell or the flow speed is measured while the centrifugal force acts on the measuring cell.

21. Method according to one of Claims 15-20, wherein the centrifugal force that is applied to the measuring cell lies in a range of 50-2000 × g.

## Revendications

1. Cellule (1, 2, 3, 4, 5, 6) de mesure pour déterminer la fonction thrombocytaire comprenant
a. une première chambre (11, 21, 31, 41 51, 61) de réception d'un échantillon liquide contenant des thrombocytes,
b. une deuxième chambre (12, 22, 32, 42, 52, 62), qui recueille l'échantillon liquide de la première chambre, dans la mesure où une force centrifuge s'applique à la cellule de mesure et
c. un élément (13, 23, 33, 43, 53, 63) de séparation poreux, qui sépare la première et la deuxième chambre l'une de l'autre,
**caractérisée en ce que**
l'élément (13, 23,33, 43, 53, 63) de séparation contient au moins une substance soluble influençant l'activité thrombocytaire.

2. Cellule de mesure suivant la revendication 1, dans laquelle la au moins une substance soluble, influençant l'activité thrombocytaire, est un activateur de thrombocytes, choisi de préférence dans le groupe ADP, 2-MeSADP, collagène, épinéphrine, ristocétine, thrombine, TRAP, acide arachidonique, U46619, PMA et sérotonine.

3. Cellule de mesure suivant la revendication 1, dans laquelle la au moins une substance soluble, influençant l'activité thrombocytaire, est un inhibiteur de thrombocytes, choisi de préférence dans le groupe prostaglandine E1, prostaglandine I2, forscholine, iloprost et cicaprost.

4. Cellule de mesure suivant l'une des revendications précédentes, dans laquelle l'élément de séparation contient, en outre, des ions de chlorure de calcium.

5. Cellule de mesure suivant l'une des revendications précédentes, dans laquelle l'élément (13, 23, 33, 43, 53, 63) de séparation a une dimension de pore de 2 à 20 µm, de préférence de 5 µm.

6. Cellule de mesure suivant l'une des revendications précédentes, dans laquelle au moins une partie de la deuxième chambre (12, 22, 32, 42, 52, 62) est en un matériau transparent.

7. Cellule de mesure suivant l'une des revendications 1 à 6, dans laquelle plusieurs paires (25, 35, 45) d'électrodes pour la mesure électrique de niveau sont montées sur la deuxième chambre (12, 22, 32, 42, 52, 62).

8. Cellule (2, 3, 4, 5) de mesure suivant l'une des revendications 1 à 7, dans laquelle la deuxième chambre (22, 32, 42, 52), qui a, à partir de l'élément de séparation poreux, suivant son axe longitudinal, une moitié proximale et une moitié distale, a, à l'intérieur, un moyen (24, 34, 44, 54) de répartition du volume de la deuxième chambre en une première partie (221, 321, 421, 521) et en une deuxième partie (222, 322, 422, 522),
- dans laquelle la première partie sert au recueil et à l'acheminement du liquide échantillon, qui a traversé l'élément de séparation et
- dans laquelle la deuxième partie sert à mesurer la quantité du liquide échantillon, qui est recueilli dans la deuxième chambre et
- dans laquelle la première et la deuxième parties sont, dans la moitié distale de la deuxième chambre, en liaison entre elles, de manière à ce que le liquide échantillon recueilli et acheminé puisse passer de la première partie à la deuxième partie.

9. Cellule (2) de mesure suivant la revendication 8, dans laquelle le moyen (24) de répartition du volume de la deuxième chambre (22), en une première et en une deuxième parties (221, 222), a un plan, qui est incliné par rapport à l'élément de séparation poreux et qui va de la moitié proximale à la moitié distale de la deuxième chambre.

10. Cellule (3) de mesure suivant la revendication 8, dans laquelle le moyen (34) de répartition du volume de la deuxième chambre (32), en une première et en une deuxième parties (321, 322),
- a une première section (341), qui a un plan incliné par rapport à l'élément de séparation poreux et
- une deuxième section (342), qui suit la première section, qui s'étend parallèlement à une paroi (36) délimitant le volume de la deuxième chambre et qui forme, avec celle-ci, une structure tubulaire allant jusque dans la moitié distale de la deuxième chambre.

11. Cellule (4) de mesure suivant la revendication 8, dans laquelle le moyen (44) de répartition du volume de la deuxième chambre (42), en une première et en une deuxième parties (421, 422), est conformé en forme d'entonnoir et la partie tubulaire du moyen en forme d'entonnoir va jusque dans la moitié distale de la deuxième chambre.

12. Cellule (2, 3, 4) de mesure suivant l'une des revendications 8 à 11, dans laquelle la deuxième partie (222, 322, 422) de la deuxième chambre, qui sert à mesurer la quantité du liquide échantillon recueilli dans la deuxième chambre, a, pour la mesure électrique du niveau, de préférence plusieurs paires (25, 35, 45) d'électrodes.

13. Cellule (5) de mesure suivant l'une des revendications 1 à 6, dans laquelle la deuxième chambre (52), qui a, à partir de l'élément de séparation poreux suivant son axe longitudinal, une moitié proximale et une moitié distale, a, à l'intérieur, un moyen (54) de répartition du volume de la deuxième chambre en une première, une deuxième et une troisième parties (521, 522, 523),
- dans laquelle la première partie (521) sert au recueil et à l'acheminement du liquide échantillon, qui a traversé l'élément (53) de séparation, et dans laquelle l'acheminement du liquide échantillon s'effectue suivant l'axe longitudinal de la deuxième chambre (52), de la moitié proximale à la moitié distale et
- dans laquelle la deuxième partie (522) sert à la mesure de la vitesse d'écoulement du liquide (59) échantillon recueilli dans la deuxième chambre (52) et
- dans laquelle la troisième partie (523) sert de trop plein pour le liquide échantillon sortant de la deuxième partie (522) et*
- dans laquelle la première et la deuxième parties (521, 522) communiquent entre elles dans la moitié distale de la deuxième chambre, de manière à ce que le liquide échantillon recueilli et acheminé puisse passer de la première partie à la deuxième partie et
- dans laquelle la deuxième partie (522) a la forme d'un tube capillaire, qui s'élève de la moitié distale en direction de la moitié proximale de la deuxième chambre et qui a, à son extrémité, une ouverture (57), qui ménage une liaison avec la troisième partie (523).

14. Système (7) de détermination simultanée de la fonction thrombocytaire dans plusieurs échantillons, **caractérisé en ce qu'**il a au moins deux cellules (6) de mesure suivant l'une des revendications 1 à 13.

15. Procédé de détermination de la fonction thrombocytaire, qui comprend les stades suivants :
i. remplissage de la première chambre d'une cellule de mesure suivant l'une des revendications 1 à 13, d'un échantillon liquide contenant des thrombocytes,
ii application d'une force centrifuge à la cellule de mesure ayant l'échantillon liquide,
iii mesure de la quantité de liquide recueillie dans la deuxième chambre ou mesure de la vitesse d'écoulement du liquide échantillon dans la deuxième chambre de la cellule de mesure,
la quantité de liquide recueillie et la vitesse d'écoulement étant correlées de manière inverse à la fonction thrombocytaire.

16. Procédé suivant la revendication 15, dans lequel on détermine, photométriquement ou électriquement, la quantité de liquide recueillie dans la deuxième chambre de la cellule de mesure.

17. Procédé suivant la revendication 15, dans lequel on détermine, au moyen d'une anémométrie doppler laser, la vitesse d'écoulement du liquide échantillon dans la deuxième chambre de la cellule de mesure.

18. Procédé suivant l'une des revendications 15 à 17, dans lequel on mesure, en continu sur un laps de temps donné, la quantité de liquide recueillie dans la deuxième chambre de la cellule de mesure ou la vitesse d'écoulement.

19. Procédé suivant l'une des revendications 15 à 17, dans lequel on mesure, une fois à un instant donné, la quantité de liquide recueillie dans la deuxième chambre de la cellule de mesure ou la vitesse d'écoulement.

20. Procédé suivant l'une des revendications 15 à 19, dans lequel on mesure la quantité de liquide recueilli dans la deuxième chambre de la cellule de mesure ou la vitesse d'écoulement, pendant qu'une force centrifuge est appliquée à la cellule de mesure.

21. Procédé suivant l'une des revendications 15 à 20, dans lequel la force centrifuge, appliquée à la cellule de mesure, est dans une plage de 50 à 2.000 x g.
